**Europäisches Patentamt**

**European Patent Office**

**Office européen des brevets**

(19)

(11) Veröffentlichungsnummer: **0 166 920**
**B1**

(12)

# EUROPÄISCHE PATENTSCHRIFT

(45) Veröffentlichungstag der Patentschrift:
20.07.88

(21) Anmeldenummer: **85105659.8**

(22) Anmeldetag: **08.05.85**

(51) Int. Cl.⁴: **A 61 M  1/14**

(54) **Einrichtung zur Entziehung von unbehandelter und behandelter Dialysierflüssigkeit und/oder Blut aus einer Dialysiervorrichtung.**

(30) Priorität: **08.05.84  DE 3416956**

(43) Veröffentlichungstag der Anmeldung:
**08.01.86 Patentblatt 86/2**

(45) Bekanntmachung des Hinweises auf die Patenterteilung:
**20.07.88 Patentblatt 88/29**

(84) Benannte Vertragsstaaten:
**DE FR GB IT**

(56) Entgegenhaltungen:
**EP-A-0 097 366**

(73) Patentinhaber: **Fresenius AG, Gluckensteinweg 5,
D-6380 Bad Homburg v.d.H. (DE)**

(72) Erfinder: **Metzner, Klaus, Dipl.- Phys., Alolfstrasse
17b, D-6380 Bad Homburg v. d. H. (DE)**
Erfinder: **Westphal, Detlef, Dr., Starenweg 1,
D-6370 Oberursel 4 (DE)**
Erfinder: **Allendörfer, Wolfgang, Dipl.- Ing. (FH),
Louisenstrasse 87, D-6380 Bad Homburg (DE)**
Erfinder: **Hahnel, Reinhard, Dipl.- Ing. (FH),
Heinrich- Schmidt- Strasse 16, D-6304 Lollar (DE)**
Erfinder: **Polaschegg, Hans- Dietrich, Dr.,
Grünwiesenweg 9, D-6370 Oberursel 4 (DE)**

(74) Vertreter: **Luderschmidt, Wolfgang, Dr. Dipl-
Chem., Görtz, Dr. Fuchs, Dr. Luderschmidt
Patentanwälte Sonnenberger Strasse 100
Postfach 26 26, D-6200 Wiesbaden (DE)**

## Beschreibung

Die Erfindung betrifft eine Einrichtung zur Entziehung von unbehandelter und behandelter Dialysierflüssigkeit und/oder Blut aus einer Dialysiervorrichtung, nach dem Oberbegriff des Anspruchs 1.

Eine derartige gattungsgemäße Einrichtung ist beispielsweise aus der DE-A-32 23 051 bekannt. Die Dialysiervorrichtung dieser bekannten Einrichtung weist einen Dialysator auf, von dem stromauf eine erste Leitung und stromab eine zweite Leitung abgeht. Diese beiden Leitungen sind zu einer dritten Leitung zusammengeführt, die mit einer Analysenvorrichtung verbunden ist. Die erste und zweite Leitung sind jeweils mit einem Absperrorgan sowie mit wenigstens einer Pumpe zur Förderung der Flüssigkeit in den Leitungen versehen.

Hierbei ist jedoch zunächst nachteilig, daß bei Stillegung einer Leitung durch Schließung des entsprechenden Absperrorgans ein Flüssigkeitsrest in der Leitung verbleibt, der bei Flüssigkeiten, wie insbesondere Blut, mangels Bewegung zur Pfropfenbildung neigt, was die Leitung verstopft. Ferner ergibt sich aus der Anordnung der Absperrorgane in unmittelbarer Nähe der Hauptleitung der Nachteil, daß bei Öffnung eines der Absperrorgane, falls eine Messung von unbehandelter oder behandelter Flüssigkeit durchgeführt werden soll, diese einen langen Strömungsweg bis zur Analysenvorrichtung zu durchfließen hat, da die Pumpe in der dritten Leitung angeordnet ist und somit die zu untersuchende Flüssigkeit praktisch unmittelbar aus der relativ weit entfernten Hauptleitung zur Analysenvorrichtung fördern muß. Daraus ergeben sich unerwünscht lange Verzögerungen, bis ein Analysenzyklus eingeleitet werden kann.

Es ist daher Aufgabe der Erfindung, eine Einrichtung zur Entziehung von unbehandelter und behandelter Dialysierflüssigkeit und/oder Blut aus einer Dialysevorrichtung, der im Oberbegriff des Anspruchs 1 umrissenen Gattung zu schaffen, die sowohl die Vermeidung von Verstopfungen in den zur Analysenvorrichtung führenden Leitungen als auch die Vermeidung unerwünscht langer Förderzeiten der zu untersuchenden Flüssigkeit zur Analysenvorrichtung ermöglicht.

Diese Aufgabe wird durch die kennzeichnenden Merkmale des Anspruchs 1 gelöst.

Durch das Vorsehen von je einer Pumpe Stromauf der Absperrorgane in der ersten und der zweiten Leitung und je eines Überlaufes zwischen Pumpen und Absperrorganen wird zum einen erreicht, daß die in den Leitungen befindliche Flüssigkeit stets im Umlauf gehalten werden kann, was ohne weitere Hilfsmittel eine Propfenbildung in den Flüssigkeiten und damit eine Verstopfung der Leitungen verhindert. Zum anderen wird durch diese Maßnahmen jedoch auch erreicht, daß die zu analysierende Flüssigkeit in unmittelbarer Nähe der Analysenvorrichtung ständig auf aktuellem Stand vorliegt, so daß nach Öffnen des jeweiligen Absperrorganes vom Überlauf bis zur Analysenvorrichtung lediglich nur noch ein kurzer Strömungsweg zurückzulegen ist, so daß eine Analyse ohne große Zeitverzögerung kurz nach Öffnen des jeweiligen Absperrorganes durchgeführt werden kann.

Die Unteransprüche haben vorteilhafte Weiterbildungen der Erfindung zum Inhalt.

Weitere Einzelheiten, Merkmale und Vorteile der Erfindung ergeben sich aus nachfolgender Beschreibung von Ausführungsbeispielen anhand der Zeichnung.

Es zeigt:

Fig. 1    eine erste Ausführungsform der erfindungsgemäßen Einrichtung,

Fig. 2    eine zweite Ausführungsform der Einrichtung gem. Fig. 1 und

Fig. 3    eine dritte Ausführungsform der Einrichtung gem. Fig. 1.

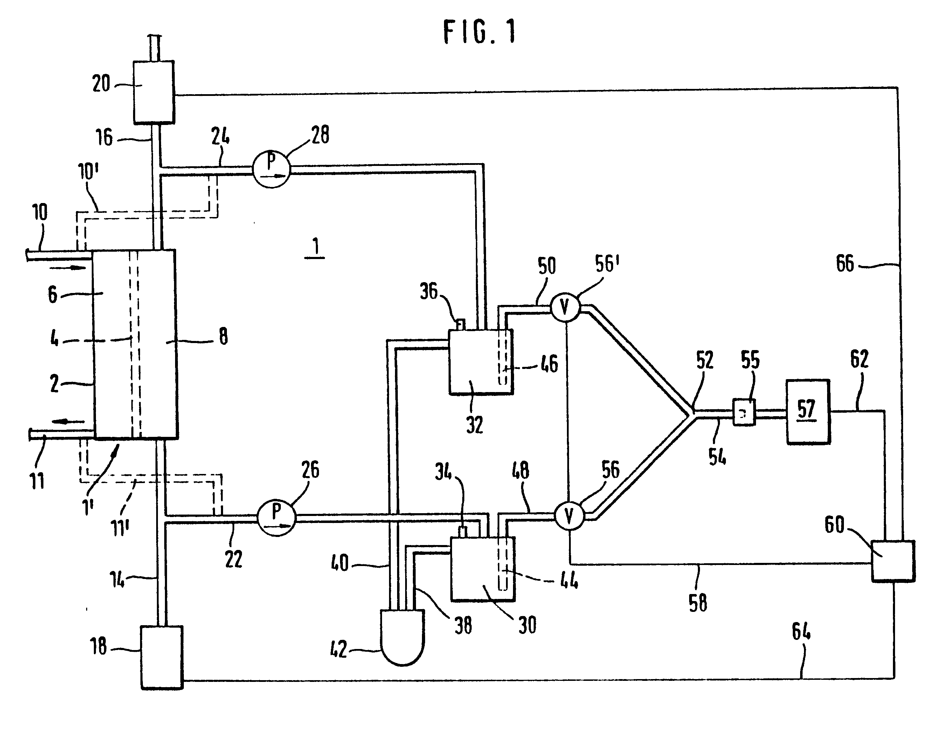

In Fig. 1 ist eine Einrichtung 1 zur Entziehung von unbehandelter und behandelter Dialysierflüssigkeit und/oder Blut aus einer Dialysevorrichtung 1' dargestellt.

Die Dialysevorrichtung 1' besteht im wesentlichen aus einem Dialysefilter 2, das eine semipermeable Membran 4 aufweist, die den Dialysefilter in zwei voneinander getrennte Kammern 6 und 8 teilt.

Die Kammer 6 weist eine Zuleitung 10 und eine Ableitung 11 für eine erste Flüssigkeit, vorzugsweise Blut, auf, während die zweite Kammer 8 eine Zuleitung 14 und eine Ableitung 16 für eine zweite Flüssigkeit, vorzugsweise für die Dialyseflüssigkeit, aufweist.

Dabei ist die Zuleitung 14 mit einer Einrichtung 18 zur Bereitstellung von frischer Dialysierflüssigkeit verbunden, während die Ableitung 16 stromab des Dialysefilters 2 mit einer Einheit 20 zur Förderung der Dialysierflüssigkeit durch den Dialysefilter 2 und zur Erzeugung eines Ultrafiltrats verbunden ist. Diese Einheit 20 kann auch aufgeteilt sein in eine reine Fördereinrichtung und in eine Ultrafiltratpumpe.

Gemäß der in Fig. 1 dargestellten Ausführungsform zweigen jeweils unmittelbar vor und hinter dem Dialysefilter 2 eine erste und eine zweite Leitung 22 bzw. 24 ab, in die jeweils Pumpen 26 bzw. 28 eingeschaltet sind, die vorzugsweise peristaltische Schlauchpumpen sind.

Die Pumpen 26 und 28 können hierbei jedoch auch als Doppelschlauchpumpe ausgeführt sein, die jeweils die Leitung 26 und die Leitung 28 mit Pumpendruck beaufschlagt.

Die Leitungen 22 und 24 münden jeweils in eine Tropfkammer 30 bzw. 32, die vorzugsweise miniaturisiert ausgebildet sind und ein Fassungsvolumen von etwa 0,2 bis 1 ml aufweisen. Diese Tropfkammern 30 und 32 weisen jeweils Entlüftungsstutzen 34 bzw. 36 auf der Oberseite auf und können hierdurch auf Normaldruck gehalten werden. Weiterhin zweigen in der Nähe des oberen Randes jeweils Überlaufleitungen 38 bzw. 40 ab, die in einem Beutel 42 münden.

In den Tropfkammern 30 und 32 sind bis in die Nähe des Bodens Rohrstücke 44 bzw. 46 eingeführt, die außerhalb der Tropfkammern 30 und 32 mit Leitungen 48 bzw. 50 in Verbindung stehen, die bei 52 vereinigt sind und in eine dritte Leitung 54 übergehen. Diese Leitung 54 ist in einen Adapter 55 eingesetzt. Dieser Adapter 55 ist wiederum Bestandteil einer Analysenvorrichtung 57.

Die Leitungen 48 und 50 können alternierend durch je ein Absperrorgan 56 bzw. 56' verschlossen bzw. geöffnet werden, so daß entweder die Tropfkammer 30 oder die Tropfkammer 32 mit der Analysenvorrichtung 57 verbunden ist.

Die im Beispielsfalle als Doppelklemme ausgebildeten Absperrorgane 56 und 56' sind über eine Leitung 58 mit einem Steuergerät 60 verbunden, daß seinen Takt über eine Leitung 62 von der Analysenvorrichtung 57 erhält.

Vorteilhafterweise ist das Totvolumen, das durch die Lumina der schlauchförmigen Leitungen 48, 50 und 54 gebildet wird, möglichst klein und beträgt im Beispielsfalle etwa höchstens 0,2 bis 0,3 ml. Bei der Analyse durch die Analysenvorrichtung 57 wird dies dadurch berücksichtigt, daß eine Pumpe der Analysenvorrichtung 57 entsprechend lange betätigt wird, so daß jeweils die unmittelbar in der Tropfkammer 30 bzw. 32 befindliche Flüssigkeit in einen Meß- oder Durchflußkanal der Analysenvorrichtung 57 gelangt und dort gemessen wird.

Wie bereits vorstehend erläutert, ist das gesamte System durch die Entlüftungsstutzen 34 und 36 druckausgeglichen, so daß keine Unterdrücke auftreten, die den Betrieb der Analysenvorrichtung 57 stören können.

Die Pumpen 26 und 28 fördern stetig Dialysierflüssigkeit aus dem Dialysatkreislauf, der durch die Leitungen 14 und 16 gebildet wird. Dabei spielt die Länge der Leitungen 22 und 24 keine wesentliche Rolle. Die Pumprate selbst liegt vorzugsweise in einem Bereich von 0,1 bis 1 ml/Minute und hat im wesentlichen keinen Einfluß auf die Ultrafiltrationsrate der Dialysevorrichtung 1.

Die gem. Fig. 1 dargestellte Einrichtung wird folgendermaßen betrieben:

Die Pumpen 26 bzw. 28 fördern stetig frische bzw. verbrauchte Dialysierflüssigkeit aus den Leitungen 14 bzw. 16 in die Tropfkammern 30 bzw. 32. Dabei ist sichergestellt, daß zum Zeitpunkt der Abförderung der in den Tropfkammern 30 und 32 enthaltenen Flüssigkeit immer eine ausreichende Menge Flüssigkeit zur Verfügung steht, was durch den Überlauf in den Beutel 42 sichergestellt wird. Die Analysenvorrichtung 57 zieht wechselweise die unbehandelte oder behandelte Dialysierflüssigkeit durch eine entsprechende Steuerung der als Doppelklemme ausgebildeten Absperrorgane 56 und 56' an, die im übrigen auch in Form von zwei separaten Klemmen vorliegen können.

Die in der Analysenvorrichtung 57 festgestellten Parameter werden dort angezeigt und können zur Steuerung der Einrichtung 18 für die Bereitstellung der Dialysierflüssigkeit und/oder der Einrichtung 20 zur Erzeugung der Ultrafiltration und zum Umpumpen der Dialysierflüssigkeit herangezogen werden. Hierzu sind die Einrichtungen 18 und 20 über Leitungen 64 bzw. 66 mit dem Steuergerät 60 verbunden.

Dementsprechend kann also die Einheit 18 veranlaßt werden, eine Dialysierflüssigkeit anderer Zusammensetzung zu erzeugen, beispielsweise eine Dialysierflüssigkeit mit erhöhtem oder erniedrigtem Natriumionengehalt, sofern am Ausgang des Dialysefilters 2 ein gesenkter oder erhöhter Natrium-Spiegel auftritt.

Andererseits kann jedoch aber auch die Ultrafiltrationseinrichtung 20 unmittelbar über die Konzentration der Ionen in der Dialysierflüssigkeit gesteuert werden, wenn man voraussetzt, daß die Diffusion der in der Analysenvorrichtung 57 gemessenen Ionen durch die Membran 4 vernachlässigt werden kann, also ein Gleichgewicht der Ionen auf beiden Seiten der Membran 4 vorliegt. Durch das Ultrafiltrat wird dabei die Konzentration der Ionen in der Dialysierflüssigkeit stromab des Dialysefilters 2 geringer, mit der Folge, daß mit der Konzentration die Ultrafiltrationsmenge bestimmt und gesteuert werden kann.

Die in Fig. 1 dargestellte Ausführungsform der erfindungsgemäßen Einrichtung 1 läßt sich natürlich auch zur Relativmessung in Blut verwenden. Dieses wird dadurch ermöglicht, daß die Leitungen 22 und 24 nicht mit den Leitungen 14 und 16, sondern vielmehr mit den in Fig. 1 gestrichelt dargestellten Leitungen 10' und 11' verbunden sind.

In einem solchen Fall wird durch die gesamte Anordnung Blut entnommen, das ebenfalls in der Analysenvorrichtung 57 bezüglich seiner Ionenkonzentration und Leitfähigkeit gemessen werden kann. Auch die hier erhaltenen Parameter können zur Steuerung der Einrichtung 18 und/oder 20 herangezogen oder aber auch auf einem nicht gezeigten Display der Analysenvorrichtung 57 sichtbar gemacht werden.

Im übrigen kann die Messung mit hoher Geschwindigkeit erfolgen, d.h. nach etwa ein bis drei Minuten kann jeweils eine Probe der Dialysierflüssigkeit bzw. des Bluts entnommen werden, während die in der anderen Tropfkammer enthaltene Flüssigkeit in den Überlaufbehälter oder Beutel überläuft, so daß grundsätzlich eine Flüssigkeit mit aktueller Zusammensetzung für die Messung zur Verfügung steht und diese ohne große Zeitverzögerung der Analysenvorrichtung 57 zugeführt werden kann. Darüber hinaus wird durch das stetige Fließen der Flüssigkeiten in der ersten und zweiten Leitung 22 und 24, was durch die ständig laufenden Pumpen 26 und 28 sichergestellt wird, eine Propfenbildung und damit eine Verstopfung der Leitungen 22 bzw. 24 in allen Betriebszuständen verhindert.

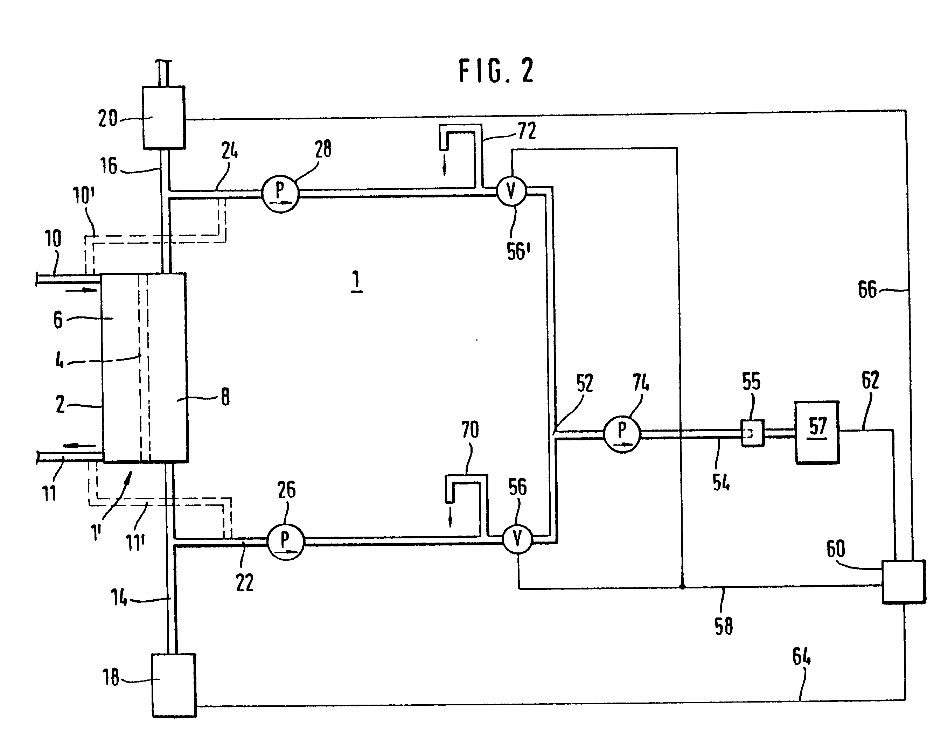

Gemäß Fig. 2 ist eine zweite Ausführungsform der erfindungsgemäßen Einrichtung 1 dargestellt, bei der alle Teile, die denjenigen der Ausführungsform gem. Fig. 1 in Aufbau und Funktion entsprechen, mit den gleichen Bezugsziffern bezeichnet sind. Im Unterschied zu der Ausführungsform gem. Fig. 1 weist diejenige gem. Fig. 2 jeweils einen Überlauf 70 bzw. 72 in der Leitung 22 bzw. 24 auf, der vor den Absperrorganen 56 bzw. 56' angeordnet ist. Diese Konstruktion ist vorteilhaft, da sie einen besonders einfachen Aufbau ermöglicht. Hinter dem Zusammenführungspunkt 52 der Leitungen 22 und 24 ist eine weitere Pumpe 74 angeordnet, die eine geringere Förderrate hat als die Förderrate der Pumpen 26 und 28. Damit wird sichergestellt, daß stets ein Flüssigkeitsüberlauf über die Überläufe 70 und 72 erfolgt, womit wiederum verhindert wird, daß Luft in das System angesaugt wird.

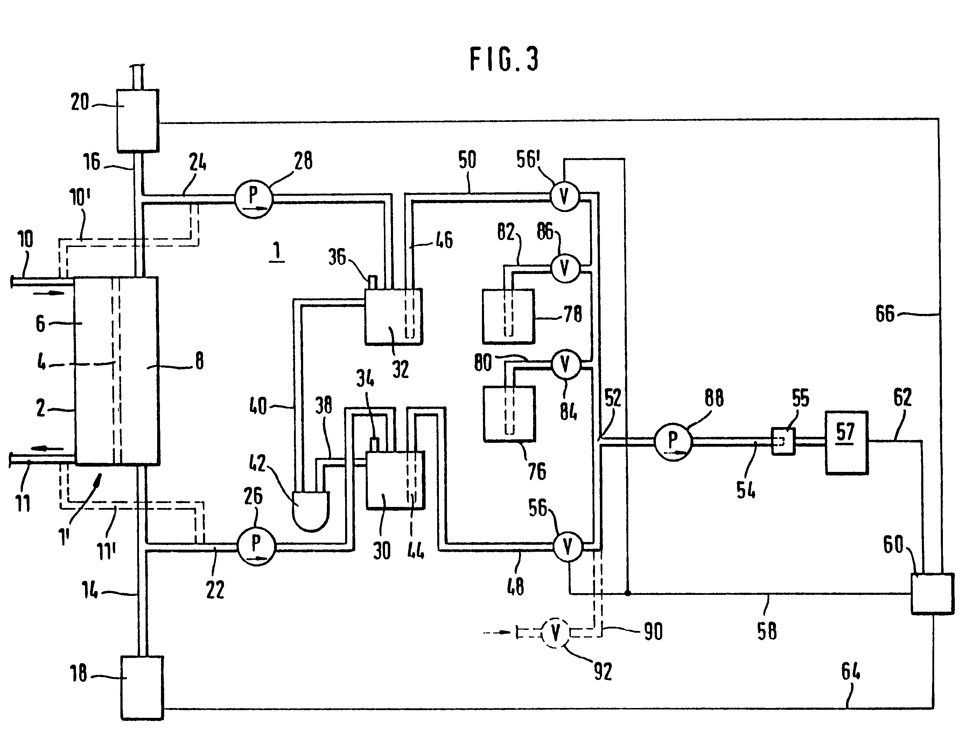

Gem. Fig. 3 ist eine dritte Ausführungsform der erfindungsgemäßen Einrichtung dargestellt, bei der wiederum alle der Fig. 1 entsprechenden Teile mit gleichen Bezugszeichen versehen sind.

Diese Ausführungsform weist zwei zusätzliche Kalibrierlösungsbehälter 76 und 78 auf, die über Leitungen 80 bzw. 82 an die Leitungen 20 bzw. 24 angeschlossen sind. In jeder der Leitungen 80 und 82 ist ein steuerbares Absperrorgan 84 bzw. 86 eingebaut. Hinter dem Zusammenführungspunkt 52 der Leitungen 22 und 24 ist eine Pumpe 88 angeordnet.

Ferner kann zusätzlich die gestrichelt eingezeichnete Leitung 90 vorgesehen sein, die an die Leitung 22 oder auch an eine andere Stelle angeschlossen sein kann, und über die Luft in das System eingesaugt werden kann. Hierfür ist ebenfalls in der Leitung 90 ein steuerbares Absperrorgan 92 eingebaut.

Der Sinn und Zweck dieser Anordnung besteht darin, daß es vorteilhaft ist, das Elektrodensystem der Analysenvorrichtung 57 mit konstanter Durchflußgeschwindigkeit und ohne Unterbrechung der Flüssigkeitssäule zu betreiben, damit das Elektrodensystem möglichst stabil arbeitet. Um hierbei eine Kalibrierung des Nullpunktes und der Steilheit des Elektrodensystems möglich zu machen, sind die Kalibrierungsbehälter 76 und 78 an den beschriebenen Stellen vorgesehen, und über die Absperrorgane 84 und 86 mit dem Zusammenführungspunkt 52 verbunden. Die hinter dem Zusammenführungspunkt 52 befindliche Pumpe 88 fördert Kalibrier- oder Meßlösung durch den Elektrodenblock der Analysenvorrichtung 57 in einen nicht näher dargestellten freien Auslauf.

Bei der Anwendung in der Hämodialyse kann davon ausgegangen werden, daß die Dialysierflüssigkeit stromauf des Dialysators in ihrer Zusammensetzung gut bekannt ist. Diese Dialysierflüssigkeit kann somit zur Kalibrierung des Nullpunktes herangezogen werden. Ohnehin wird nur eine Relativmessung beabsichtigt. Im allgemeinen ist ein Mischsystem einer Dialysemaschine sehr viel stabiler als eine ionenselektive Elektrode. Die Steilheit der Elektroden kann vor Beginn der Dialyse getestet werden, indem die Konzentration in der Dialysierflüssigkeit gezielt verändert wird. Sie kann ferner immer dann geprüft werden, wenn aufgrund eines Regelvorganges die Konzentration der Dialysierflüssigkeit während der Hämodialyse verändert wird. Dies kann z. B. wie folgt geschehen:

Vor Beginn der Dialyse wird das Hämodialysegerät mit einem Konzentrat bestimmter Elektrolytzusammensetzung in Betrieb genommen. Sobald sich ein stabiler Konzentrationszustand in der Dialysierflüssigkeit eingestellt hat, was durch einen üblicherweise vorhandenen Monitor im Hämodialysegerät festgestellt werden kann, wird stromauf des Dialysators Flüssigkeit entnommen und gewartet, bis sich an den ionenselektiven Elektroden der Analysenvorrichtung 57 ein stabiles Potential eingestellt hat. Dieses wird abgelesen und als Nullpunkt gespeichert. Anschließend wird die Konzentration der Dialysierflüssigkeit durch entsprechende Steuerung des Mischsystems des Hämodialysegerätes um einen bestimmten Betrag erhöht. Sobald an den ionensensitiven Elektroden wieder ein stabiler Zustand herrscht, wird ein zweiter Potentialwert abgelesen und aus der Differenz dieses Potentialwertes gegenüber dem Nullpunktwert sowie der Konzentrationsdifferenz die Steilheit erreichnet. Damit sind die ionensensitiven Elektroden nach Nullpunkt und Steilheit hin kalibriert.

## Patentansprüche

1. Einrichtung zur Entziehung von unbehandelter und behandelter Dialysierflüssigkeit und/oder Blut aus einer Dialysiervorrichtung, die einen Dialysator (2) aufweist, von dem stromauf eine erste Leitung (22) und stromab eine zweite Leitung (24) abgeht, die zu einer dritten Leitung (54) zusammengeführt sind, die mit einer Analysenvorrichtung (57) verbunden ist, mit jeweils einem Absperrorgan (56, 56') in der ersten und der zweiten Leitung sowie mit wenigstens einer Pumpe zur Förderung der Flüssigkeiten in den Leitungen, dadurch gekennzeichnet, daß jeweils in der ersten und der zweiten Leitung (22, 24) jeweils eine Pumpe (26, 28) Stromauf der Absperrorgane (56, 56') vorgesehen ist, und daß in jeder der Leitungen (22, 24) zwischen den Pumpen (26, 28)

und den Absperroganen (56, 56') ein Überlauf (38, 40) angeordnet ist.

2. Einrichtung nach Anspruch 1, dadurch gekennzeichnet, daß. die Pumpen (26, 28) als Doppelschlauchpumpe ausgebildet sind.

3. Einrichtung nach Anspruch 1 oder 2, dadurch gekennzeichnet, daß in der ersten und der zweiten Leitung (22, 24) je eine druckausgeglichene Tropfkammer (30, 32) angeordnet ist.

4. Einrichtung nach Anspruch 3, dadurch gekennzeichnet, daß die Tropfkammern (30, 32) mit den Überläufen (38, 40) versehen sind.

5. Einrichtung nach einem der Ansprüche 1 - 4, dadurch gekennzeichnet, daß die Überläufe als Überlaufleitungen (38, 40) ausgebildet sind, die mit einem Auffangbeutel (42) verbunden sind.

6. Einrichtung nach Anspruch 3, dadurch gekennzeichnet, daß in die Tropfkammern (30, 32) jeweils Rohrstücke (44, 46) geführt sind, von denen Leitungen (48, 50) abzweigen, die bei einem Vereinigungspunkt (52) unter Bildung der dritten Leitung (54) vereinigt sind, die mit einem Adapter (55) verbunden sind.

7. Einrichtung nach Anspruch 6, dadurch gekennzeichnet, daß die Absperrorgane (56, 56') als wechselweise betätigbare Klemmeinrichtung ausgebildet sind und in den von den Rohrstücken (44, 46) abzwigenden Leitungen (48, 50) angeordnet sind.

8. Einrichtung nach einem der Ansprüche 1 - 7, dadurch gekennzeichnet, daß die Absperrorgane (56, 56') und die Analysenvorrichtung (57) durch ein Steuergerät (60) betätigt sind.

9. Einrichtung nach einem der Ansprüche 1 - 8, dadurch gekennzeichnet, daß hinter dem Vereinigungspunkt (52) der ersten und zweiten Leitung (22, 24) eine weitere Pumpe (74) in der dritten Leitung (54) angeordnet ist, deren Förderrate geringer ist als diejenige der Pumpen (26, 28).

10. Einrichtung nach einem der Ansprüche 1 - 9, dadurch gekennzeichnet, daß vor dem Vereinigungspunkt (52) der ersten und zweiten Leitung (22, 24) Kalibrier- oder Meßlösung enthaltende Kalibrierlösungsbehälter (76, 78) angeordnet sind, die über zugeordnete Absperrorgane (84, 86) mit den ersten und zweiten Leitungen (22, 24) verbunden sind, und daß hinter dem Vereinigungspunkt (52) eine Pumpe (88) zur Förderung der Kalibrier- oder Meßlösung in der dritten Leitung (54) angeordnet ist.

**Claims**

1. An apparatus for drawing off of untreated and treated dialyzing liquid and/or blood from a dialysis device which has a dialyzer (2) from which a first line (22) goes off upstream and a second line (24) goes off downstream which join into a third line (54) which is connected to an analysis device (57), with a shut-off element (56, 56') in both the first and the second line and with at least one pump for conveying the liquids in the lines, characterized in that respectively in the first and the second line (22, 24) respectively one pump (26, 28) is provided upstream of the shut-off element (56, 56') and that in each of the lines (22, 24) an overflow (38, 40) is arranged between the pumps (26, 28) and the shut-off elements (56, 56').

2. Apparatus according to claim 1, characterized in that the pumps (26, 28) are designed as double-tube pumps.

3. Apparatus according to claim 1 or 2, characterized in that a pressure-equalizing drop chamber (30, 32) is arranged in the first and the second line (22, 24).

4. Apparatus according to claim 3, characterized in that the drop chambers (30, 32) are provided with overflows (38, 40).

5. Apparatus according to one of claims 1 - 4, characterized in that the overflows are designed as overflow lines (38, 40) which are connected to a collecting bag (42).

6. Apparatus according to claim 3, characterized in that into the drop chambers (30, 32) respectively pipe pieces (44, 46) are passed from which lines (48, 50) branch off which join at a junction point (52) forming the third line (54) which are connected to an adapter (55).

7. Apparatus according to claim 6, characterized in that the shut-off elements (56, 56') are designed as alternately operable clamp mechanism and are arranged in the lines (48, 50) branching off the pipe pieces (44, 46).

8. Apparatus according to one of claims 1 - 7, characterized in that the shut-off elements (56, 56') and the analysis device (57) are actuated by a control unit (60).

9. Apparatus according to one of claims 1 - 8, characterized in that a further pump (74) is arranged behind the junction point (52) of the first and second line (22, 24) in the third line (54) whose conveying rate is less than that of the pumps (26, 28).

10. Apparatus according to one of claims 1 - 9, characterized in that calibrating solution containers (76, 78) containing calibrating or measuring solution are arranged ahead of the junction point (52) of the first and second line (22, 24) and which are connected via associated shut-off elements (84, 86) to the first and second lines (22, 24) and that a pump (88) for conveying the calibrating or measuring solution is arranged behind the junction point (52) in the third line (54).

**Revendications**

1. Installation pour l'extraction de liquide de dialyse, non traité ou traité et/ou de sang d'un dispositif de dialyse présentant un dialyseur (2) duquel partent un premier conduit (22) en amont et un deuxième conduit (24) en aval, lesquels sont réunis dans un troisième conduit (54) relié à un dispositif d'analyse (57), avec respectivement un organe de retenue (56, 56') dans le premier et le second conduit et au moins une pompe pour le transport des liquides dans les conduits,

caractérisée en ce qu'une pompe (26, 28) est respectivement prévue en amont des organes de retenue (56, 56') dans chacun des premier et deuxième conduits et qu'un trop-plein (38, 40) est disposé dans chacun des conduits (22, 24) entre les pompes (26, 28) et les organes de retenue (56, 56').

2. Installation selon la revendication 1, caractérisée en ce que les pompes (26, 28) se présentent sous forme de pompe tubulaire double corps.

3. Installation selon l'une des revendication 1 ou 2, caractérisée en ce qu' une chambre goutte à goutte (30, 32) équilibrée en pression est disposée dans chacun des premier et deuxième conduits (22, 24).

4. Installation selon la revendication 3 caractérisée en ce que les chambres goutte à goutte (30, 32) sont pourvues des trop-pleins (38, 40).

5. Installation selon l'une des revendications 1 à 4, caractérisée en ce que les trop-pleins se présentent sous forme de conduits de trop-plein (38, 40) reliés à un réceptacle (42).

6. Installation selon la revendication 3 caractérisée en ce que des pièces tubulaires (44, 46), desquelles dérivent des conduits (48, 50) sont respectivement introduites dans les chambres goutte à goutte, lesdits conduits étant réunis en un point de jonction (52) pour former un troisième conduit 54, relié à un adaptateur (55).

7. Installation selon la revendication 6 caractérisée en ce que les organes de retenue (56, 56') se présentent sous forme d'un dispositif de serrage pouvant être actionné alternativement et sont disposés dans les conduits (48, 50) dérivant des pièces tubulaires (44, 46).

8. Installation selon l'une des revendications 1 à 7, caractérisée en ce que les organes de retenue (56, 56') et le dispositif d'analyse (57) sont actionnés par un appareil de commande (60).

9. Installation selon l'une des revendications 1 à 8, caractérisée en ce qu'une autre pompe (74) dont le taux d'alimentation est inférieur à celui des pompes (26, 28) est disposée dans le troisième conduit (54), derrière le point de jonction (52) du premier et du deuxième conduit (22, 24).

10. Installation selon l'une des revendications 1 à 9, caractérisée en ce que des récipients de standardisation (76, 78) contenant de la solution standard ou de la solution de mesure sont disposés avant le point de jonction (52) du premier et du deuxième conduit (22, 24) et sont reliés à ces conduits au moyen d'organes de retenue (84, 88) qui leur sont adjoints et qu'une pompe (88) pour le transport de la solution standard ou de mesure est disposée derrière le point de jonction (52) dans le troisième conduit (54).

FIG. 1

0 166 920

FIG. 2

# FIG.3

0 166 920